# EUROPEAN PATENT APPLICATION

(11) **EP 1 164 374 A1**
(43) Date of publication of application: **19.12.2001**
(21) Application number: 00112813.1
(22) Date of filing: 16.06.2000
(51) Int. Cl.: G01N 33/561, G01N 33/68, C07K 14/705

(54) **Method for identifying apoptosis-modified proteins**

(71) Applicant: Max-Planck-Gesellschaft zur Förderung der Wissenschaften e.V., 80539 München (DE)
(72) Inventor: Rudel, Thomas Dr., 13591 Berlin (DE); Thiede, Bernd Dr., 10823 Berlin (DE)
(74) Representative: Weiss, Wolfgang, Dipl.-Chem. Dr.

(57) **Abstract**

The present invention relates to a method for characterizing or identifying apoptosis-modified proteins which are expressed by cells, preferably human cells. Further, novel apoptosis-modified proteins are provided which are suitable as targets for diagnosis, prevention or treatment of diseases, particularly hyperproliferative or degenerative diseases.

## Description

The present invention relates to a method for characterizing and/or identifying apoptosis-modified proteins which are expressed by cells, preferably mammalian cells, more preferably T-cells, most preferably human T-cells. Further, novel apoptosis-modified proteins are provided which are suitable as targets for diagnosis, prevention or treatment of diseases, particularly hyperproliferative or degenerative diseases.

Apoptosis is an essential and complex process for the development and homeostasis of multicellular organisms. Improper regulation of this process results in various diseases including cancer, autoimmune disorders, viral infections, neurodegenerative disorders and myocardial infarction (1). The therapeutic regulation of apoptosis therefore offers numerous challenges (2).

Several components of the apoptotic cell death machinery were already identified. The best known contributors are the caspases (3,4) and their inhibitors (5) and substrates (6), the bcl-2 family (7,8), the death receptors (9), the mitochondria (10,11) and signal transduction pathways (12,13). Death receptors belong to the tumor necrosis factor (TNF) superfamily. The best characterized death receptors are Fas, TNFR1, DR3, DR4 and DR5. These receptors induce apoptosis by ligand binding and receptor oligomerization, recruitment of an adaptor protein to the death domain of the receptor. The adaptor molecule binds a caspase, thereby activating the apoptosis machinery. On the other hand decoy receptors compete with specific death receptors for ligand binding.

However, hundreds of stimuli induce apoptosis independent of death-receptor like UV or γ-irradiation, chemotherapeutic drugs and viral or bacterial infections. The apoptotic phenotype is very similar in all apoptotic cells independent of the stimuli used to induce apoptosis. In addition, apoptosis of cells from organisms which are evolutionary distantly related, like nematodes and man, is regulated by structurally related proteins like caspases and these cells show similar phenotypes. These findings together were the basis for a concept of a highly conserved apoptotic machinery involving similar factors in all cells.

The Fas receptor (CD95 or Apo1) plays an important role in immune regulation by deletion of autoimmune cells and activation-induced T-cell death, killing of targets such as virus-infected cells or cancer cells by cytotoxic T-cells and by natural killer cells and killing of inflammatory cells at immune privileged sites (14-16). Fas is expressed in a wide variety of cells, whereas the Fas ligand (FasL) has a limited tissue distribution. FasL is rapidly induced in activated T-cells and natural killer cells but few other cells appear to express significant levels of FasL. The decoy receptor DcR3 binds to FasL and inhibits FasL-induced apoptosis (17). Thus, tumours may be able to evade the death signal by binding of a trigger of apoptosis.

Proteome approaches have been used to find new apoptosis-associated proteins (18). However, the conditions used in these studies to induce apoptosis allowed synthesis of new proteins because (1) protein synthesis was not blocked by the addition of protein synthesis inhibitors such as cycloheximide and (2) the cells were stimulated to undergo apoptosis for such a long time (more than 12 h) that synthesis of new proteins was possible. The modified proteins obtained by this treatment thus consisted of apoptosis-modified proteins and proteins which were expressed as a general response of the cell to stress. The identification of a protein as apoptosis-modified was thus not possible.

Thus, the object underlying the present invention was to provide a method allowing characterization or identification of apoptosis-modified proteins, which does not suffer from the disadvantages as described above.

In order to solve this problem we induced apoptosis by the addition of Anti-Fas IgM antibody in a defined way for 6 h and the same time blocked the synthesis of new proteins by the addition of cycloheximide. Under these conditions only apoptosis-modified proteins, and not newly synthesised proteins, were detected. This is also very important for the apoptosis-induced translocation of proteins which can be attributed to the movement of a pre-formed protein upon apoptosis induction. Translocation from the cytosol to the nucleus in apoptotic cells of the pre-formed caspase activated DNAse (CAD) is shown in (33).

Thus, the present invention provides a proteome analysis of cells to characterize and/or identify apoptosis-modified proteins. Subtractive analysis of two dimensional gel-electrophoresis patterns of apoptotic cells and non-apoptotic cells revealed differences in a plurality of protein spots. The predominantly altered protein spots were identified after proteolytic digestion and peptide mass fingerprinting. Of the identified proteins, the heterogeneous nucleoprotein (hnRNP) A/B, hnRNP A2/B1, hnRNP A3, hnRNP D, hnRNP F, hnRNP H, hnRNP I, hnRNP K, hnRNP L, hnRNP R and hnRNP JKTBP1, the splicing factors SRp30c, P54nrb, SF2p33 (ASF-2), SF SC35, NMP200 (related to SR PRP19) and PTB-associated SF, the translation factors EF-Tu, EF-1 beta and EIF-5A, the factors involved in signal transduction GAP SH3 binding protein and cGMP-dependent protein kinase, the chromatins type I alpha, Baf-57 and CAF-1 (RB b.p.) (WD-repeats), the transcription factor CBF-beta, the proteasomal factor 26S protease SU 12, proteasome subunit C8 and Tat binding protein-1, the mitchondrial factors isocitrate dehydrogenase and AOP-1, and the diverse factors SYT interacting protein SIP, PA1-G, CRHSP-24, HCD2, GMP synthase, FUSE binding protein 1 and HDGF were hitherto unknown to be involved in apoptosis. HnRNP C1/C2, nucleolin, p54nrb, Rho GDI2, ASF-2, SRp30c and BTF3 include aspartic acid/glutamic acid-rich domains and hn RNP A2/B1, hn RNP C1/C2, nucleolin and BTF3 interact with protein kinase CK2. Remarkably, the heterogeneous nuclear ribonucleoprotein (hnRNP) A/B, hnRNP A1, hnRNP A2/B1, hnRNP A3, hnRNP C1/C2, hnRNP D, hnRNP F, hnRNP H, hnRNP I, hnRNP K, hnRNP L, hnRNP R, hnRNP JKTB1, the splicing factors SRp30c, P54nrb, SF2p33 (ASF-2), SFSC35, and PTB-associated SF, the signal transduction protein GAP SH3 binding protein, and the chromatin associated protein nucleolin contain the RNP motif.

'Modification' or 'apoptosis-modified' in this context describes the alteration of a protein in a given compartment during the process of apoptosis. The protein spot elicits changes in the size or the charge or the size and the charge. 'Translocation' in this context describes differences in the localisation of a protein in compartments of apoptotic cells compared to the compartments of non-apoptotic cells.

The method established and described above can be used for other cell types expressing death receptors like TNF-receptor, DR-3, DR-4 or DR-5 or any receptor which induces apoptosis in the absence of protein biosynthesis. The method can be used for cells induced to undergo apoptosis by other pathways than the receptors described above.

Thus, a subject matter of the present invention is a method for characterizing and/or identifying apoptosis-modified proteins comprising the steps:
(a) providing a first extract and a second extract comprising soluble proteins, wherein said first extract is from a cell without apoptosis induction and said second extract is from a cell after apoptosis induction,
(b) separating said first and second extracts by two-dimensional gel electrophoresis, wherein said first and second proteome patterns each comprising a plurality of protein species are obtained
(c) comparing said first and second proteome patterns and
(d) characterizing and/or identifiying apoptosis-modified protein species.

In the context of the present application, characterization of a protein is the analysis of the chemical composition of the protein. Identification of a protein is the assignment of a spot on the 2-DE gel to its biological functions or at least the assignment to a gene including the regulatory encoding sequences. In the context of the present invention, the proteome comprises the protein composition of a cell or a part of it at a defined biological situation (19).

The method of the present invention allows characterization and identification of apoptosis-modified proteins from cells, preferably from mammalian cells, more preferably from human cells, such as mammalian and particularly human T-cells, e.g. from an immortalized T-cell line such as the T-cell line Jurkat E6 (ATCC TIB 152).

Step (a) of the method of the invention comprises the preparation of extracts comprising soluble proteins. A first extract is obtained from a cell without apoptosis induction and a second extract is obtained from a cell after apoptosis induction. The extracts may be whole cell extracts but may be also extracts from cell compartments such as membranes, cytosol, mitochondria or nucleus. Apoptosis may be induced by contacting the cells with caspase activators and/or ligands of death receptors such as an anti-Fas antibody.

Preferably, the second extract is obtained from a cell wherein after apoptosis induction substantially no synthesis of new proteins has been allowed. This may be effected by adding an inhibitor of protein biosynthesis such as cycloheximide and/or by carrying out apoptosis induction for a period of time which is too short to allow a substantial synthesis of new proteins, e.g. a period of time of less than 12 h, preferably less than 8 h, e.g. about 6 h.

Step (b) of the method of the invention is a two-dimensional gel electrophoresis which comprises (i) separation in a first dimension according to the isoelectric point and (ii) separation in a second dimension according to size. The gel matrix is preferably a polyacrylamide gel. Gel preparation may be carried out according to known methods (20,21).

Step (c) of the method of the invention comprises comparing said first and second proteome patterns. This comparison may comprise a subtractive analysis of the first and second proteome patterns (22). By means of this subtractive analysis apoptosis-modified protein species are obtained which may be selected from protein species which (i) are located at different positions on the two-dimensional gels from the first and second extracts and/or (ii) have a different intensity on the two-dimensional gels from the first and second extracts.

The characterization of apoptosis-modified protein species may be carried out by peptide fingerprinting, wherein peptide fragments of the protein to be analysed are generated by in-gel proteolytic digestion, e.g. by digestion with trypsin. Further characterization of the peptides may be carried out by mass spectrometry, e.g. electrosprayionization massspectrometry (ESI-MS) (23) and matrix-assisted laser dissorption/ionization mass spectrometry (MALDI-MS) (24) and/or by at least partial amino acid sequencing, e.g. by Edman degradation.

In a preferred embodiment, the invention further comprises as step (e) the determination if the apoptosis-associated modifications of the protein species are present in subjects, e.g. experimental animals or human patients suffering from apoptosis-associated diseases including hyperproliferative or degenerative diseases such as cancer, autoimmune and neurodegenerative disorders such as Alzheimer's disease, viral infections such as AIDS and vascular diseases such as myocardial infarction. By screening the presence of apoptosis-modified proteins in the patients, valuable targets for preventing or treating the above diseases may be identified.

A further subject matter of the present invention are proteomes from an apoptotic T-cell or a compartment thereof consisting of a pattern of individual proteins obtainable by the method as described above. The proteins consist of highly resolved patterns of proteins, comprising preferably at least 100, more preferably at least 500 and most preferably at least 1.000 different protein species, which are expressed by apoptotic T-cells. The term "protein species" describes a chemically clearly defined molecule in correspondance to one spot on a high performance 2-DE pattern. Preferably, the proteomes of the present invention, which may be in the form of two-dimensional gel electrophoresis pictures or electronic data bases thereof (25,26,27), contain the proteins as shown in Table 1 or at least a part thereof.

A still further subject matter of the present invention are individual proteins which are expressed by apoptotic cells, e.g. by apoptotic T-cells, and which have been characterized and identified by the method as described above. Preferably, these proteins are selected from heterogenous nuclear ribonucleoproteins such as hnRNP A/B (Gene bank Accession Number NM_004499), A1 (X12671), A2/B1 (D28877), A3 (AF148457), C1/C2 (NM_004500), D (D55671), F (L28010), H (L22009), I (NM_002819), K (NM_002140), L (NM_001533), R (AF000364) and JKTBP1 (D89092), splicing factors such as SRp30c (NM_003769), p54nrb (U89867), SF2p33 (ASF-2) (M72709), SFSC35 (X62447), NMP200 (AJ131186) and PTB-associated SF (NM_05066), translational factors such as 60S acidic ribosomal protein (NM_001002), EF-Tu (NM_003321), EF-1β(NM_001959) and EIF-5A (NM_001970), structural proteins such as lamin B1 (L37747), lamin B2 (M94362), vimentin (NM_003380) and beta-tubulin (V00599), signal transduction proteins such as GAP SH3 binding protein (NM_005754), Rho GDI2 (X69549), cGMP-dependent protein kinase type Iα (Z92867), chromatin associated proteins such as nucleolin (NM_005381), Baf-57 (NM_003079), CAF-1 (X71810), transcription factors such as BTF3 (X53281) and CBF-β (L20298), proteasome subunits such as 26S protease subunit12 (NM_002811), proteasome subunit C8 (NM_002788) and Tat binding protein-1 (NM_02804), mitochondrial proteins such as isocitrate dehydrogenase (NM_002168) and AOP-1 (NM_006793), nucleophosmin (X16934), SYT interacting protein SIP (NM_006328), PA1-G (NM_002573), CRHSP-24 (AF115345), HCD2 (NM_004493), GMP synthase (NM_003875), FUSE binding protein 1 (NM_003902) and HDGF (NM_004494). More preferably, these proteins are selected from the proteins as shown in Table 1.

In addition to the proteins as specified above or fragments thereof having a length of preferably at least 10, more preferably at least 20 and most preferably at least 30 amino acids, the invention also relates to nucleic acids, e.g. DNA, RNA or nucleic acid analogs, e.g. DNA which encode these proteins or protein fragments or variants, e.g. allelic variants thereof. Further, the invention relates to substances capable of modulating the characteristics of the proteins or nucleic acids, e.g. antibodies, low molecular weight inhibitors or activators, antisense molecules or ribozymes.

The proteins or protein patterns as described above may be used as targets for the diagnosis, prevention or treatment of apoptosis-associated diseases or in a method for identifying apoptosis-modulators. A diagnostic method may comprise a determination of the presence or absence of apoptosis-modified proteins in a sample. A preventive or therapeutic method may comprise the activation or inhibition of apoptosis-modified proteins, e.g. an activation by overexpression via gene transfer into cells or organs by gene transfer vectors such as viruses, an inhibition by antisense or ribozyme molecules or an activation or inhibition by substances which modulate the amount, processing, presentation or conformation of the protein. The method for identifying apoptosis modulators (activators or inhibitors) may comprise a screening assay, e.g. a cellular or molecular screening assay which may be carried out in a high-throughput format.

The proteins described in this application or proteins identified by the method described above can be used to develop modification-specific diagnostic tools such as antibodies or phages or other substances. The proteins or useful fragments can be used to develop protein chips or other solid-phase screening devises for high throughput screens.

The proteins identified by this technique are potential targets for diseases associated with apoptosis. Such diseases are tumours which can be associated with identified proteins as GAP SH3 binding protein (NM_005754), Baf-57 (4507089), CAF-1 (422892), CBF-beta (2498753), AOP-1 (5802974), SYT interacting protein SIP (5454064), PA1-G (4505587), CRHSP-24 (4583307), FUSE binding protein 1 (4503801) and HDGF (4758516). Further diseases are viral infections like HIV infection which can be associated with identified proteins as Tat binding protein-1 (4506211), CBF-beta (2498753) and EIF-5A (4503477). Further diseases are neurodegenerative diseases like Alzheimer's disease and Parkinson's disease which can be associated with identified proteins as HCD2 (4758504) and AOP-1 (5802974). Further diseases are ischemic stroke, heart failure and arthritis, which can be associated with identified protein AOP-1 (5802974).

Therefore the lack of expression or over-expression can be indicative of a disease and thus has diagnostic implications. The genes of the identified proteins can be used to develop DNA-chips or other DNA-or RNA-based screening devices (PCR, RT-PCR) to screen cells or tissues for the differences in the mRNA levels of the identified genes.

GAP SH3 binding protein or fragments generated during apoptosis can be used to generate diagnostic tools such as cleavage specific antibodies or phages or other tools useful for large scale screening. The gene of the GAP SH3 binding protein can be used to develop DNA-chips or other DNA- or RNA-based screening devices (PCR, RT-PCR) to screen cells or tissues for the differences in the mRNA levels of the identified genes or to screen for mutations in the caspase cleavage site of the GAP SH3 binding protein.

GAP SH3 protein or fragments generated during apoptosis can be used to screen drugs which activate or inhibit their activity. This activity may be modification of the activity of Ras-GAP which modifies the activity of the Ras-oncoprotein or other GTPases. The activity may be the RNA-binding or RNAse activity elicted by the apoptosis-specific modification of GAP SH3 binding protein. This activity may be any activity elicited by the modification of the protein during apoptosis.

GAP SH3 binding protein is therefore potentially involved in the growth control of cells. Tumours can over-express or lack GAP SH3 binding protein or produce a modified GAP SH3 binding protein. Tumours can be defective in the RNA-modifying activity of GAP SH3 binding protein. Drugs which interfere with constitutive GAP SH3 binding protein activity or which activate GAP SH3 binding protein activity are useful for therapy of such diseases.

Alzheimer's disease is associated with premature apoptosis of neuronal cells. Neuronal cells of Alzheimer patients are characterised by the accumulation of β-amyloid precursor protein which is known to interact with HCD2 (Yan et al., 1997, Nature, 389, 689-695). HCD2 was found to translocate from the cytosol to the nucleus (compare Tables 4 and 5) and is thereby modified, probably by phosphorylation. HCD2 translocation can be the cause of β-amyloid precursor accumulation and thus a promoter of Alzheimer's disease.

HCD2 or the modified HCD2 generated during apoptosis can be used to generate diagnostic tools such as modification-specific antibodies or phages or other tools useful for large scale screening. The gene of the HCD2 protein can be used to develop DNA-chips or other DNA- or RNA-based screeing devices (PCR, RT-PCR) to screen cells or tissues for the differences in the mRNA levels of the identified genes or to screen for mutations in the modification site (phosphorylation site) of the HCD2 protein.

HCD2 or the modified HCD2 generated during apoptosis can be used to screen drugs which activate or inhibit their activity and which are useful in prevention and/or treatment of Alzheimer's disease. This activity can be binding and/or sequestration of the β-amyloid precursor protein and prevention of apoptosis in neuronal cells or other cells. The activity can be the enzymatic activity of the HCD2 which is preferably any activity associated with prevention of apoptosis and more preferably a dehydrogenase activity (34). This activity can be any activity elicited by the modification or/and translocation of the protein during apoptosis.

AOP-1 protects radical-sensitive proteins (enzymes) from oxidative damage. Oxidative stress has been demonstrated to induce apoptosis in different cell types. In addition, oxidative stress is involved in several diseases. AOP-1 as protecting molecule can be used to prevent and/or to treat diseases related to oxidative stress like ischemic stroke, arthritis, heart failure, Parkinson's disease, Alzheimer's and amyotrophic lateral sclerosis (ALS). The cleavage and/or translocation of AOP-1 (see Table 5) from the mitochondria to the nucleus is accompanied with a change in its activity. AOP-1 or the modified AOP-1 generated during apoptosis can be used to generate diagnostic tools such as modification-specific antibodies or phages or other tools useful for large scale screening. The gene of the AOP-1 protein might be used to develop DNA-chips or other DNA- or RNA-based screening devices (PCR, RT-PCR) to screen cells or tissues for the differences in the mRNA levels of the identified genes or to screen for mutations in the modification site (cleavage site) of the AOP-1 protein.

AOP-1 or the modified AOP-1 generated during apoptosis can be used to screen drugs which modify their activity. This activity can be protection from radical induced damage of proteins and therapy of the diseases outlined above. The activity can be the enzymatic activity of the AOP-1 which is preferably any activity associated with prevention of apoptosis and more preferably a peroxide reductase activity. This activity can be any activity elicited by the modification or/and translocation of the protein during apoptosis. The gene of the AOP-1 protein can be used for gene therapy of diseases associated with radical induced protein damage followed by apoptosis.

The present invention is to be further illustrated by the following figures and examples.

### Figure 1

2-DE gel of Fas-induced Jurkat T-cells (see Table 3).

### Figure 2

2-DE gel of Jurkat T-cells (control, see Table 3).

### Figure 3

2-DE gel of the cytosolic fraction of Fas-induced Jurkat T-cells (see Table 4).

### Figure 4

2-DE gel of the cytosolic fraction of Jurkat T-cells (control, see Table 4).

### Figure 5

2-DE gel of the nucleic fraction of Fas-induced Jurkat T-cells (see Table 5).

### Figure 6

2-DE gel of the nucleic fraction of Jurkat T-cells (control, see Table 5).

### Figure 7

2-DE gel of the mitochondrial fraction of Fas-induced Jurkat T-cells (see Table 6)

### Figure 8

2-DE gel of the mitochondrial fraction of Jurkat T-cells (control, see Table 6)

### Figure 9

Peptide mass fingerprinting of unknown protein called PFC6D1 (see Table 3). The peptide is characterized by fragments with the following masses: 1462.01, 1477.9, 1484.9, 1550.11, 1615.04, 2529.33, 2543.22 dalton.

### Figure 10

Peptide mass fingerprinting of unknown protein called KPF1 (see Table 5). The peptide is characterized by fragments with the following masses: 842.15, 992.529, 1006.57, 1092.58, 1109.6, 1274.68, 1288.68, 1265.76, 1249.58, 1338.73, 1455.74, 1564.74, 1758.93, 2004.03, 2034.09, 2080.96, 2110.75, 2211.09 and 2250.33 dalton.

### Figure 11

Peptide mass fingerprinting of unknown protein called KNFE3 (see Table 5). The peptide is characterized by fragments with the following masses: 696.42, 967.438, 1060.59, 1252.67, 1289.72, 1310.65, 1417.79, 1554.92, 1582.9, 1594.75, 1640.73, 1649.76, 1979.94, 1994.05 dalton.

### Figure 12

The partial sequence TPGT(F/Mox)E of the protein KNFE3 was obtained by ESI-MS/MS of the 1649,79 dalton fragment.

### Example

### 1. Materials and methods

### 1.1 Cell culture

The Jurkat T-cell line E6 (ATCC TIB 152) was maintained in RPMI tissue culture medium (Gibco BRL, Karlsruhe, Germany) supplemented with 10% fetal calf serum (Gibco BRL, Karlsruhe, Germany) and penicillin (100 U/ml)/streptomycin (100 *µ*g/ml) (Gibco BRL, Karlsruhe, Germany) at 37°C in 5.0% CO₂.

### 1.2 Induction of apoptosis

Apoptosis was induced to 2 x 10⁶ Jurkat T-cells for 6 h at 37°C in 5.0% CO₂ by 250 ng/ml αCD95 (clone CH11) (Immunotech, Marseille, France). 10 *µ*g/ml cycloheximide was added to the control- and the Fas induced cells.

### 1.3 2-DE Gel Electrophoresis

The proteins were separated by a large gel 2-DE technique (gel size 30 cm x 23 cm) (28). The isoelectric focusing rod gels (diameter 1.5 mm or 2.5 mm) contained 3.5% acrylamide, 0.3% piperazine diacrylamide (Bio-Rad, Munich, Germany) and a total of 4% w/v carrier ampholytes WITAlytes pH 2-11 (WITA GmbH, Teltow, Germany). About 200 *µ*g to 500 *µ*g of protein were applied to the anodic side of the gel and focused at 8870 Vh. After focusing, the gels were equilibrated for 10 minutes in a buffer containing 125 mM Tris/phosphate, pH 6.8, 40% glycerol, 70 mM dithiothreitol (DTT), and 3% SDS. The equilibrated gels were frozen at -70°C. After thawing, the isoelectric focusing gels were immediately applied to SDS-PAGE gels, which contained 15% w/v acrylamide and 0.2% bisacrylamide. The SDS-PAGE system of Laemmli, 1970 was used, replacing the stacking gel by the equilibrated IEF gel. Electrophoresis was performed using a two-step increase of current, starting with 15 minutes at 120 mA, followed by a run of about 6 hours at 150 mA until the front reached the end of the gel.

### 1.4 Staining

### 1.4.1 Staining with Coomassie Blue R-250

Preparative gels were stained with Coomassie Brilliant Blue R-250 (Serva, Heidelberg, Germany). After fixation over night in 1 I 50% ethanol/10% acetic acid/40% water, the gel was stained for at least 5 hours in 1 I 50% methanol/10% acetic acid/40% water, 1 g Coomassie Blue R-250. The staining solution was removed and the gel was destained for 1 hour with 1 I 5% methanol/12.5% acetic acid/82.5% water. Subsequently, the gel was kept for 4 hours in aqueous 7% acetic acid and stored at 4°C in a plastic foil.

### 1.4.2 Staining with silver nitrate

Analytical gels were stained with silver nitrate. After fixation for at least one hour in 1 I 50% ethanol/10% acetic acid/40% water, the gel was incubated for 2 hours in 1 I 30% ethanol/0.5 M sodium acetate/0.5 glutaraldehyde/0.2% sodium thiosulfate. After washing with water twice for 20 minutes, the gel was stained with 1 I 0.1 % silver nitrate/0.01% formaldehyde for 30 minutes. After washing for 30 seconds, the gel was developed for at least 4 minutes in 2.5% sodium carbonate, pH 11.3/0.05 mM sodium thiosulfate/0.01 % formaldehyde. The staining process was stopped by applying 0.05 M Titriplex III/0.02% Thimerosal. The solution was renewed after 15 minutes. Finally, the gels were dried for 3 hours at 70°C between cellophane membranes using a gel dryer (Model 585, Bio-Rad, München, Germany).

### 1.5 Tryptic digestion

The Coomassie Blue R-250 stained single gel spots from Jurkat T-cells were excised with a scalpel and shrunk by addition of 100 *µ*l 50 mM ammonium bicarbonate, pH 7.8/acetonitrile (1:1) for 30 minutes at 37°C under shaking. Subsequently the solution was exchanged against 100 *µ*l 50 mM ammonium bicarbonate, pH 7.8 for reswelling of the gel piece for 30 minutes at 37°C under shaking. The gel spots were dried in a vacuum concentrator (Eppendorf, Hamburg, Germany) after removing the buffer. 0.1 *µ*g of trypsin (Promega, Madison, Wl, USA) solved in 1 *µ*l 50 mM acetic acid and 19 *µ*l 50 mM ammonium bicarbonate, pH 7.8 were added. After incubation at 37°C for 16 hours the supernatant was removed and the gel pieces were washed with 20 *µ*l 0.5% aqueous TFA/acetonitrile (2:1) and again the supernatant was removed. The combined supernatants were evaporated in the vacuum concentrator and solved in 4 *µ*l 0.5% aqueous TFA/acetonitrile (2:1) for the mass spectrometrical analysis.

### 1.6 Peptide mass fingerprinting by MALDI-MS

The mass spectra were recorded by using a time-of-flight delayed extraction MALDI mass spectrometer (Voyager-Elite, Perseptive Biosystems, Framingham, MA, USA). The samples were mixed in an Eppendorf tube with the same volume of the matrix solution. Twenty mg/ml α-cyano-4-hydroxycinnamic acid (CHCA) in 0.3% aqueous TFA/acetonitrile (1:1) or 50 mg/ml 2,5-dihydroxybenzoic acid (DHB) in 0.3% aqueous TFA/acetonitrile (2:1) were used as matrices. Two *µ*l of the mixtures were applied to a gold-plated sample holder and introduced into the mass spectrometer after drying. The spectra were obtained in the reflectron mode by summing 100-200 laser shots with the acceleration voltage of 20 kV, 70% grid voltage, 0.05 guide wire voltage, 100 ns delay and the low mass gate at 500 m/z.

### 1.7 Sequencing by ESI-MS/MS

The mass spectra were aquired with a quadrupole/time-of flight ESI mass spectrometer equipped with a nebulized nanoelectrospray Z-spray source (Q-Tof, Micromass, Manchester, GB). Therefore, the tryptic digest was purified with a ZipTip C-18 tip (Millipore, Eschborn, Germany). The sample was evaporated and then dissolved in 2 *µ*l 1 % acetic acid/49% water/50% methanol, Subsequently, 1 *µ*l was introduced in the mass spectrometer using a nanospray needle to generate the mass spectra.

### 1.8 Database searching

The proteins were identified by using the peptide mass fingerprinting analysis software MS-Fit (http://prospector.ucsf.edu/ucsfhtml3.2/msfit. htm). The NCBI database with the species human and mouse was used for the searches by considering at maximum one missed cleavage site, pyro-Glu formation at N-terminal Gln, oxidation of methionine, acetylation of the N-terminus and modification of cysteines by acrylamide.

The molecular masses and isoelectric points were calculated by employing the software Compute pl/Mw (http://www.expasy.ch/tool/pi_tool.html).

### 2. Results for the total cell Iysate

### 2.1 Identification of apoptosis-modified protein spots

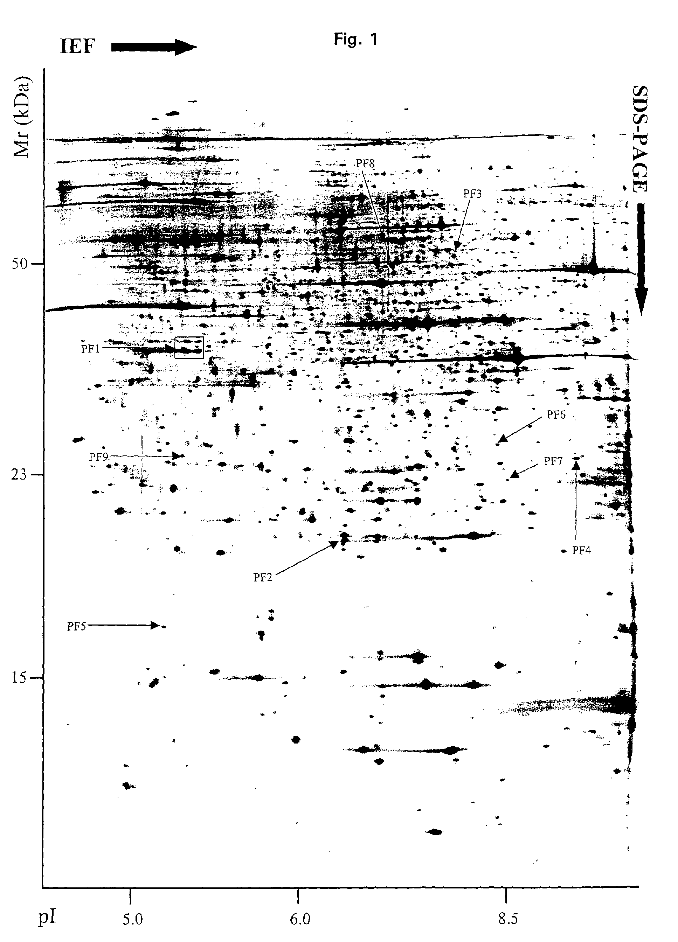

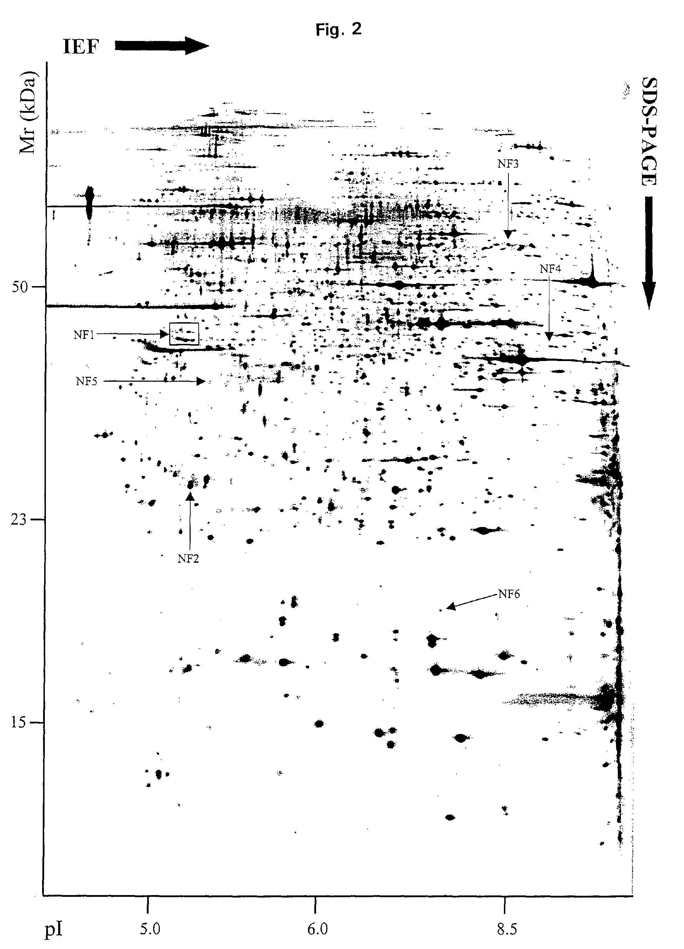

Apoptosis was induced in Jurkat T-cells by treatment with an anti-Fas antibody for six hours. 2-DE gels were produced after lysis of the cells and separation of the proteins. A representative 2-DE gel of Fas-induced Jurkat T-cells is shown in Figure 1. Approximately 2000 spots were resolved and detected by silver staining. Ten 2-DE gels of apoptotic cells were compared with ten 2-DE gels of Jurkat T-cells (Figure 2). Protein patterns of apoptosis-induced cells and control cells were found to be highly reproducible. In Fas-induced Jurkat T-cells 24 additional spots and in untreated Jurkat T-cells 21 additional spots were observed. Coomassie stained 2-DE gels were used for the identification by mass spectrometry.

### 2.2 Identified proteins

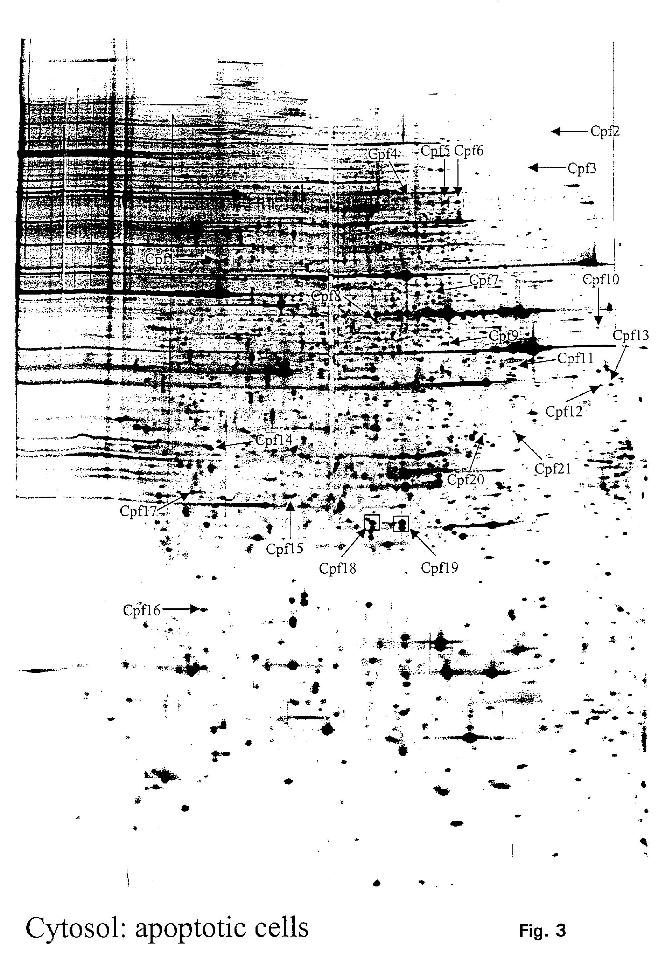

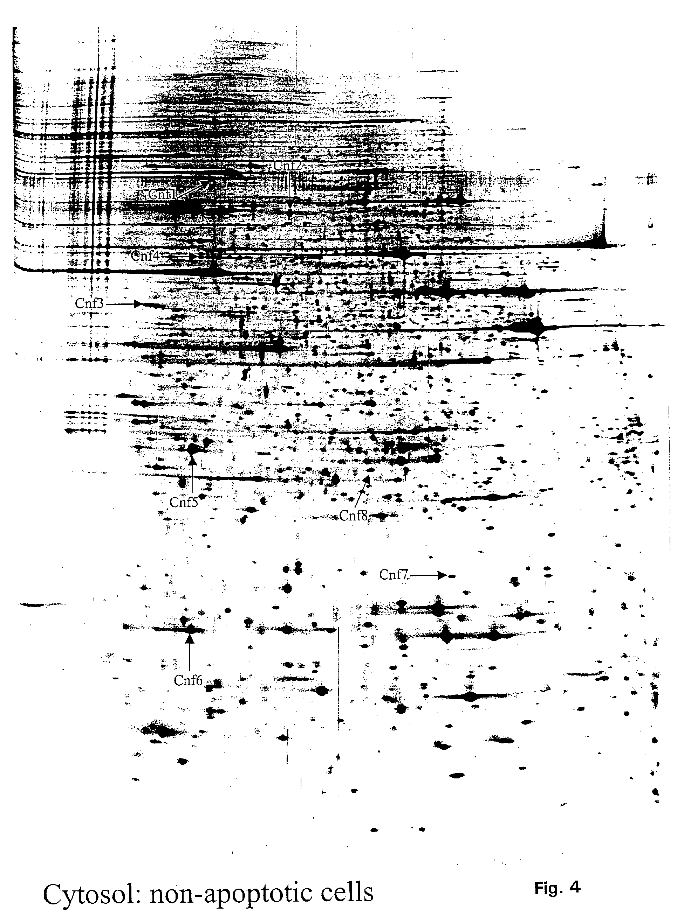

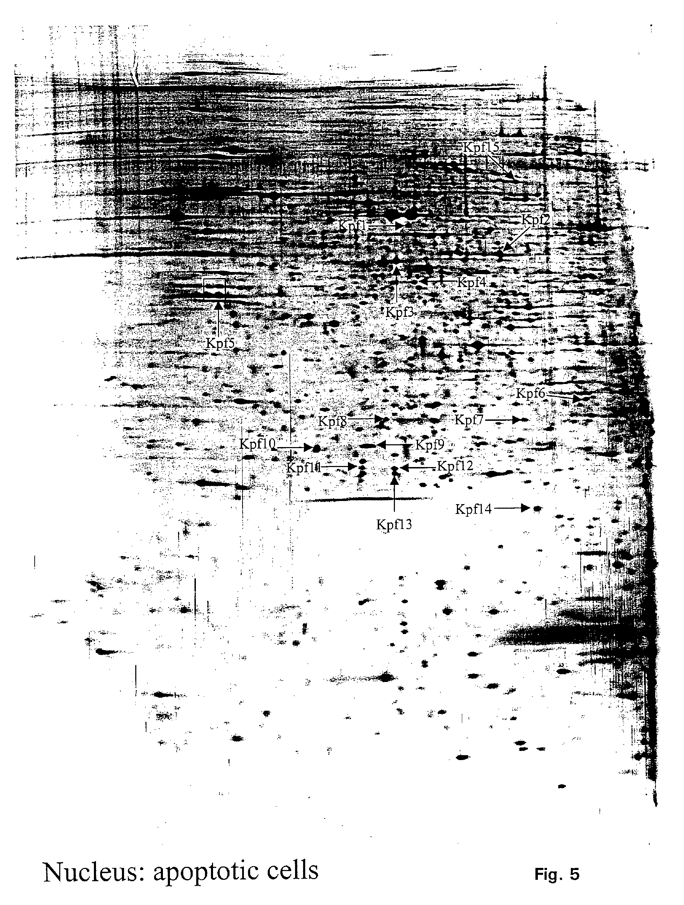

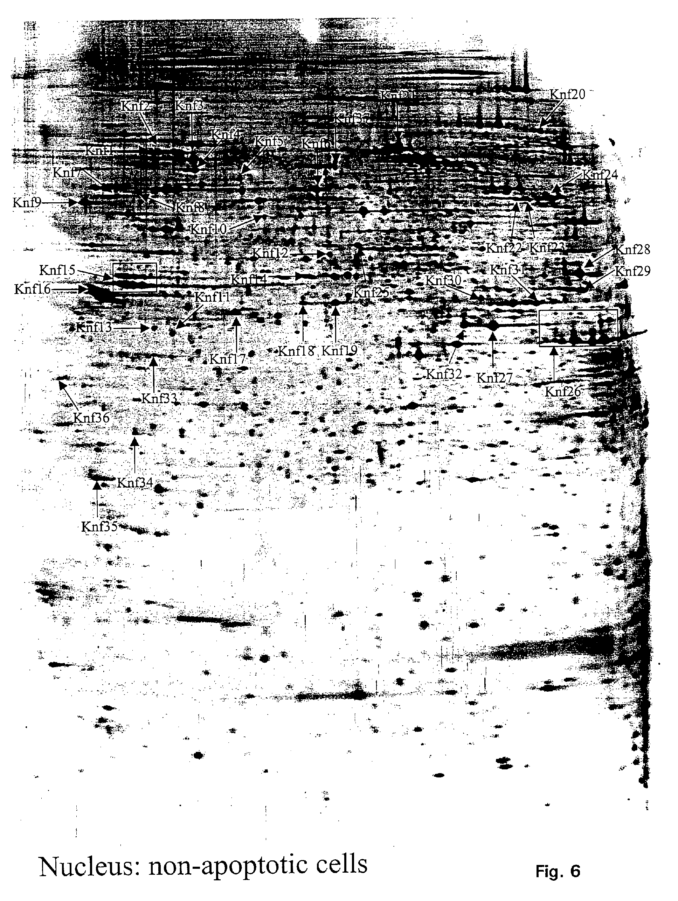

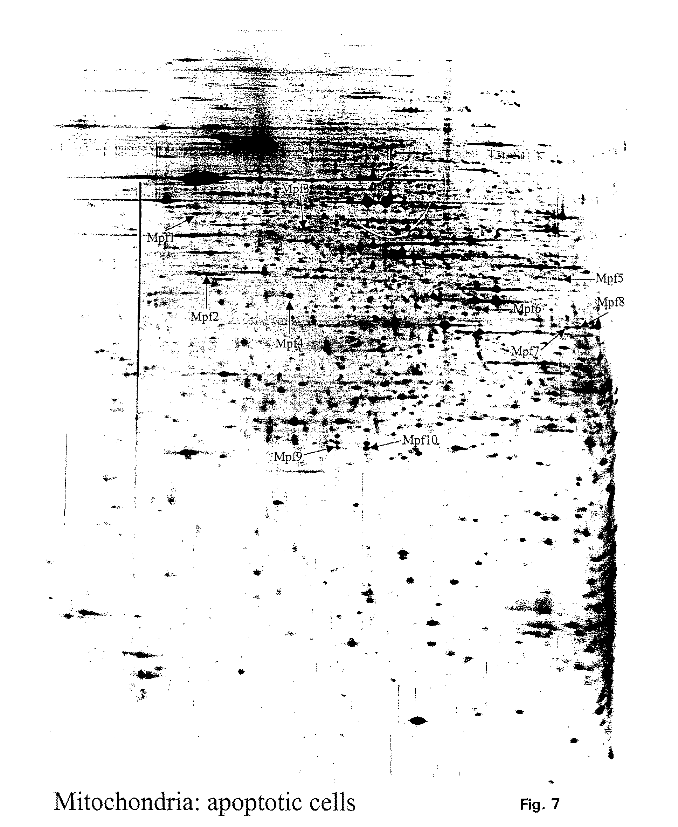

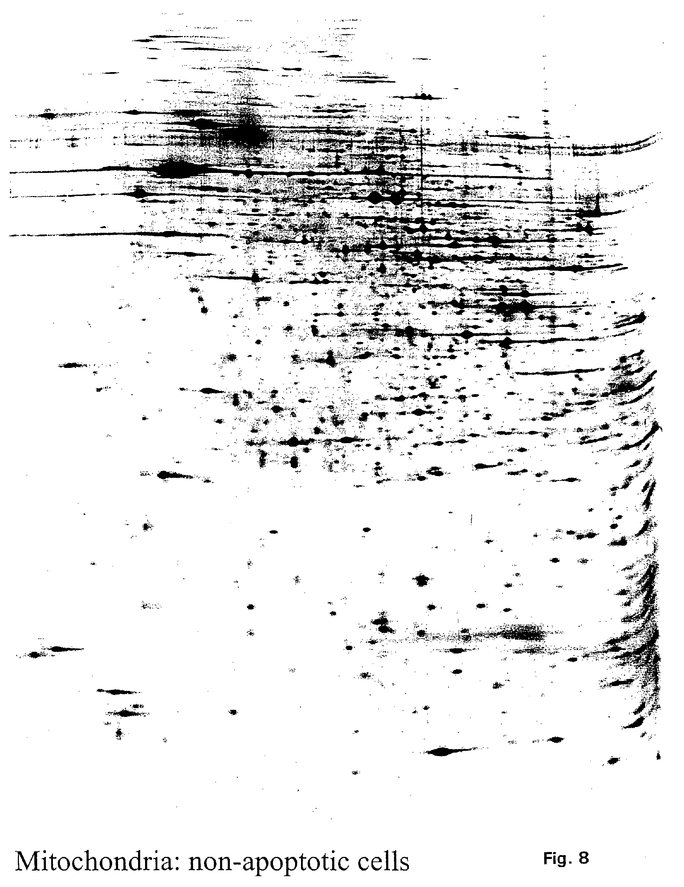

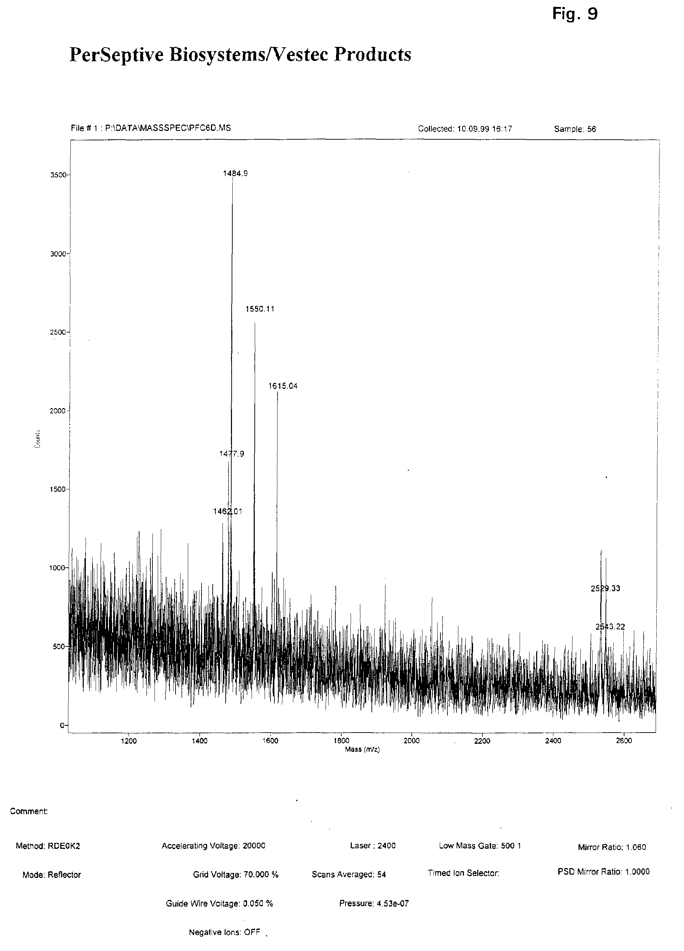

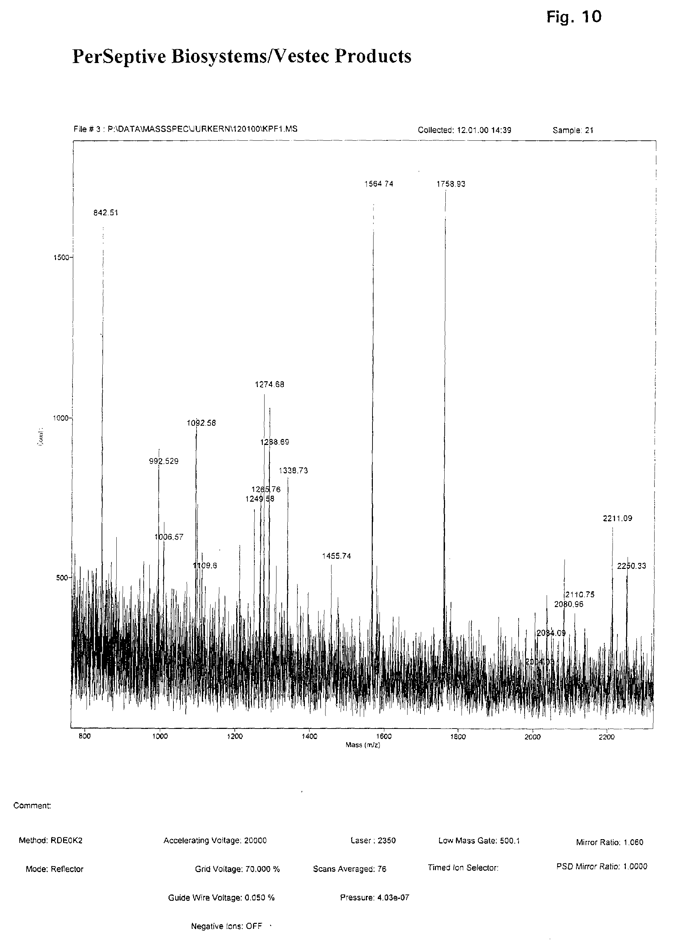

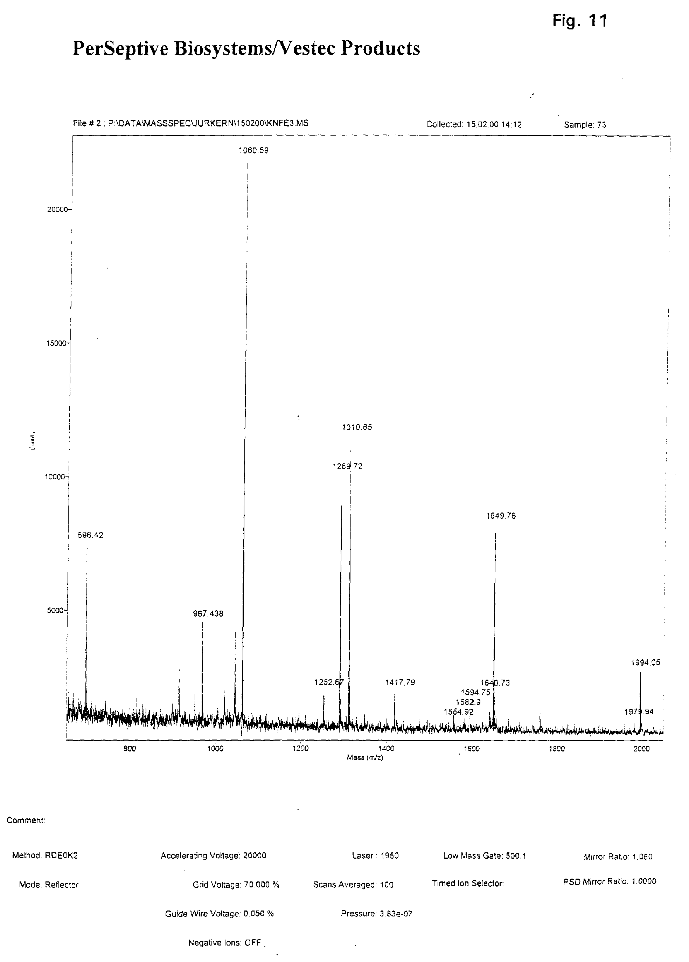

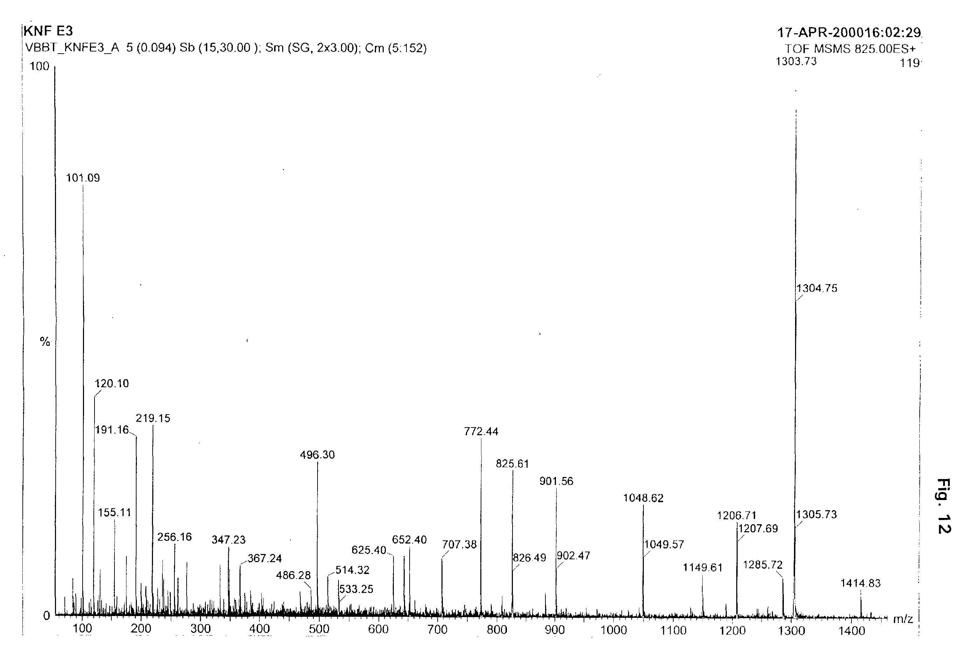

The proteins of the total cell lysate (Table 1a) within 19 spots were identified by peptide mass fingerprinting after in-gel digestion with trypsin, elution of the generated peptides and analysis by DE-MALDI-MS (figure 3). Four proteins (hnRNP A2/B1, hnRNP C1/C2, p54nrb and Rho GDI 2) were found at different spot positions in negative- and positive Fas cells, whereas the other proteins were only identified at one condition.

The molecular mass of protein spots in 2-DE gels can usually be determined with an accuracy of about 10%. The identified proteins in negative Fas gels displayed the theoretical mass of the corresponding protein. Five of the apoptosis-modified positive Fas proteins showed a significant decreased mass, whereas the remaining three proteins hnRNP C1/C2, p54nrb and splicing factor SRp30c retained the expected theoretical mass. The negative Fas spot of p54nrb showed an increased mass of 3.6 kD in comparison to the positive Fas spot of the same protein (Figure 3). The negative Fas spot of the hnRNP C1/C2 spots displayed an increased mass of 1 kD and decreased pI of 0.4 in comparison to the positve Fas spots. The mass and pl of the splicing factor SRp30c in Fas-positive Jurkat T-cells showed the theoretical values. These results indicate that predominantly cleavage events have occurred within the identified proteins during the apoptotic process.

The identified protein share similarities concerning function and motifs. The hnRNPs and the splicing factors are involved in the splicing process. 8 proteins contain the RNP-motif and 7 proteins include an aspartic acid/glutamic acid rich domain. Interaction with protein kinase CK2 was already identified for hnRNP A2/B1, hnRNP C1/C2, nucleolin and the transcription factor BTF3.

### 2.3 Prediction of cleavage sites after Fas-induction

Seven proteins were reduced in mass after Fas-induction. Considering the sequence coverage of the peptide mass fingerprint and the difference of the theoretical and the detected mass and pl lead to calculate approximately the cleavage site of the protein (Table 2). The identified protein spots of hnRNP A2/B1 and Rho GDI 2 was cleaved at the amino-terminal end, hnRNP A1, hnRNP R and p54nrb at the carboxy-terminal end and nucleolin at both sites.

The cleavage sites can be estimated more precisely by taking in account that caspases were responsible for the degradation. These enzymes cleave target proteins at specific aspartic acids. Only one cleavage site is possible for p54nrb, Rho GDI 2 and the amino-terminal cleavage of nucleolin, whereas two sites can be calculated for hnRNP A1, hnRNP A2/B1, hnRNP C1/C2 and for the carboxy-terminal cleavage of nucleolin (Table 2).

Concerning the specificities of the caspases, the most likely cleavage site for hnRNP A2/B1 is the sequence AEVD, for the carboxy-terminal cleavage of nucleolin the sequence AMED. The two possible cleavage sites of hnRNP A1 are quite equal concerning caspase specificity. Two cleavage sites can be calculated for hnRNP C1/C2 but it can be assumed likewise that the known phosphorylation may be the reason for the shift in pl, which is supported by the fact that hnRNP C1/C2 was identified in neighboring seven spots. The possible cleavage of hnRNP R was relatively difficult to calculate. Most reasonable was an amino- and carboxy-terminal cleavage which lead approximately to the found mass and pl.

The RNP consensus sequence of the RNP motif is composed of two short sequences, RNP1 and RNP2, and a number of other conserved amino acids (29). Five of the six identified shortened proteins contain one or more RNP motifs. The RNP1 and RNP2 consensus sequences of hnRNP A1, hnRNP R, p54nrb, one of the two of hnRNP A2/B1 and two of the four of nucleolin are within the sequence of the identified protein spots. No cleavage within the sequence from RNP2 to RNP1 has occurred. On the hand, the carboxy-terminal sequence in hnRNP A1, termed M9, was separated from the protein.

### 3. Discussion

Apoptosis-modified proteins were identified by a proteome approach after Fas-induction. The proteins which were found in the total cell lysate hnRNP A2/B1, hnRNP R, p54nrb, splicing factor ASF-2 and splicing factor SRp30c were not yet described to be related to apoptosis. The five proteins hnRNP A1, hnRNP C1/C2, nucleolin, Rho GDI 2 and transcription factor BTF3 were already known to be associated to apoptosis. These proteins were identified as well by a proteome approach in the human Burkitt Lymphoma cell line HL60 after IgM-mediated apoptosis (18,30,31). However, hnRNP A1, nucleolin and Rho GDI 2 were identified at other spot positions compared to the Jurkat T-cells. These results prove that the proteome approach can be useful to identify apoptosis-modified proteins at different experimental conditions.

More than 60 substrates for caspases have been already described (6). These proteins can be activated or inactivated due to the cleavage. The caspase substrates are involved in different processes e.g. cell cycle, replication, transcription, translation, DNA cleavage, DNA repair and function as kinases, cytoskeletal and structural proteins. The results of this study indicated that cleavage events have occurred within the identified proteins, probably by caspases.

The most striking feature of the identified apoptosis-modified proteins of the total cell lysate is that eight of the proteins contain the RNP-binding motif and seven of the eight proteins, with the exception of nucleolin, are involved in the splicing process.

The RNP-motif, also known as RBD or RRM (29), was identified in about 300 proteins. It is composed of two consensus sequences, RNP2 and RNP1, and a number of other amino acids within a total length of about 90 amino acids. The three dimensional structure was solved first in the U1A spliceosomal protein. RNA-binding proteins are involved in the regulation of gene expression. In particular, the regulation of RNA by signalling allows a cell to respond much faster to a stimuli than protein expression from *de novo* transcription. Specific mRNAs can be stored as mRNA-protein complexes and in response to a stimulus the masking proteins are removed or modified and the mRNA is translated. Consideration of the identified protein spots revealed that no cleavage occurred within the RNP-motif. Hence it can be assumed that the RNA-binding properties are probably not affected by the apoptotic process.

Many proteins involved in alternative splicing contain RNA-protein binding motifs. Alternative splicing of pre-mRNA is a process for generating functionally different proteins from the same gene. The splicing reaction is catalyzed by the spliceosome, which is formed by small nuclear ribnucleoproteins (snRNPs) and a large number of splicing factors. In particular, proteins of the SR family play important roles in splicing control. Furhermore, phosphorylation modulates protein-protein interactions within the spliceosome.

An important factor for the complex regulation of apoptosis may well be pre-mRNA splicing. Alternative splicing was identified for some contributors to apoptosis. Death receptors, Bcl-2 family members, caspases and CED-4 showed alternative splice forms (32). Apoptosis-associated proteins can be generated by splicing with different functions and subcellular localization. The potential crucial role in regulation of apoptosis by splicing was confirmed strongly by the fact that the predominantly number and significance of the altered proteins were involved in splicing process.

### Reference List

1. Thompson, C.B. (1995) *Science* **267,** 1456-1462
2. Nicholson, D.W. (1996) *Nat*.*Biotechnol*. **14,** 297-301
3. Cohen, G.M. (1997) *Biochem.J.* **326,** 1-16
4. Thornberry, N.A. and Lazebnik, Y. (1998) *Science* **281,** 1312-1316
5. Miller, L.K. (1999) *Trends Cell Biol*. **9,** 323-328
6. Stroh, C. and Schulze-Osthoff, K. (1998) *Cell Death Differ.* **5,** 997-1000
7. Adams, J.M. and Cory, S. (1998) *Science* **281,** 1322-1326
8. Gross, A., McDonnell, J.M., and Korsmeyer, S.J. (1999) *Genes Dev.* **13,** 1899-1911
9. Ashkenazi, A. and Dixit, V.M. (1999) *Curr*.*Opin*.*Cell Biol*. **11,** 255-260
10. Green, D. and Kroemer, G. (1998) *Trends Cell Biol*. **8,** 267-271
11. Green, D.R. and Reed, J.C. (1998) *Science* **281,** 1309-1312
12. Guo, M. and Hay.B.A. (1999) *Curr*.*Opin*.*Cell Biol*. **11,** 745-752
13. Downward, J. (1998) *Curr.Opin.Genet.Dev.* **8,** 49-54
14. Griffith, T.S. and Ferguson, T.A. (1997) *Immunol*.*Today* **18,** 240-244
15. Nagata, S. and Golstein, P. (1995) *Science* **267,** 1449-1456
16. O'Connell, J., Bennett, M.W., O'Sullivan, G.C., Collins, J.K., and Shanahan, F. (1999) *Immunol*. *Today* **20,** 46-52
17. Pitti, R.M., Marsters, S.A., Lawrence, D.A., Roy, M., Kischkel, F.C., Dowd, P., Huang, A., Donahue, C.J., Sherwood, S.W., Baldwin, D.T., Godowski, P.J., Wood, W.I., Gurney, A.L., Hillan, K.J., Cohen, R.L., Goddard, A.D., Botstein, D., and Ashkenazi, A. (1998) *Nature* **396,** 699-703
18. Brockstedt, E., Rickers, A., Kostka, S., Laubersheimer, A., Dorken, B., Wittmann-Liebold, B., Bommert, K., and Otto, A. (1998) *J*.*Biol*.*Chem.* **273,** 28057-28064
19. Wilkins, M.R., Pasquali, C., Appel, R.D., Ou, K., Golaz, O., Sanchez, J.C., Yan, J.X., Gooley, A.A., Hughes, G., Humphery-Smith, I., Williams, K.L., and Hochstrasser, D.F. (1996) *Biotechnology (N. Y.)* **14,** 61-65
20. O'Farrell, P.H. (1975) *J*.*Biol*.*Chem*. **250,** 4007-4021
21. Klose, J. (1975) *Humangenetik* **26,** 231-243
22. Aebersold, R. and Leavitt, J. (1990) *Electrophoresis* **11,** 517-527
23. Fenn, J.B., Mann, M., Meng, C.K., Wong, S.F., and Whitehouse, C.M. (1989) *Science* **246,** 64-71
24. Karas, M. and Hillenkamp, F. (1988) *Anal*.*Chem*. **60,** 2299-2301
25. Appel, R.D., Bairoch, A., Sanchez, J.C., Vargas, J.R., Golaz, O., Pasquali, C., and Hochstrasser, D.F. (1996) *Electrophoresis* **17**, 540-546
26. Lemkin, P.F. (1997) *Electrophoresis* **18,** 461-470
27. Celis, J.E., Ostergaard, M., Jensen, N.A., Gromova, I., Rasmussen, H.H., and Gromov, P. (1998) *FEBS Lett.* **430,** 64-72
28. Klose, J. and Kobalz, U. (1995) *Electrophoresis* **16,** 1034-1059
29. Burd, C.G. and Dreyfuss, G. (1994) *Science* **265,** 615-621
30. Brockstedt, E., Otto, A., Rickers, A., Bommert, K., and Wittmann-Liebold, B. (1999) *J*.*Protein Chem.* **18,** 225-231
31. Rickers, A., Brockstedt, E., Mapara, M.Y., Otto, A., Dorken, B., and Bommert, K. (1998) *Eur*.*J*.*Immunol*. **28,** 296-304
32. Jiang, Z.H. and Wu, J.Y. (1999) *Proc*.*Soc*.*Exp*.*Biol*.*Med*. **220,** 64-72
33. Enari M, Sakahira H, Yokoyama H, Okawa K, Iwamatsu A, Nagata S: A caspase-activated DNAse that degrades DNA during apoptosis, and its inhibitor ICAD (1998) *Nature* **393,** 393-396.
34. Yan et al. (1999) *J. Biol*. *Chem.* **274,** 2145-2156.

**Table 1a**

| Several identified apoptosis-modified proteins in Jurkat T-cells. | | | | | | | | | |
|---|---|---|---|---|---|---|---|---|---|
| Protein | NCBI | Spot | Mr found | Mr theor. | pl found | pl theor. | RNP-motif | D/E-rich | CK2* |
| hnRNP A1 | 133254 | PF6 | 32100 | 38846 | 8.5 | 9.26 | 2 | - | - |
| hnRNP A2/B1 | 4504447/ | NF4PF4 | 36400 | 36006 | 9.0 | 8.67/ | 2 | - | + |
| | | | 29900 | / | | | | | |
| | 133257 | | | | 9.3 | 8.97 | | | |
| | | | | 37429 | | | | | |
| hnRNP C1/C2^{#} | 133298 | NF1 | 36300 | 33298 | 5.0 | 5.11 | 1 | + | + |
| | | | | | | | | | |
| | | PF1 | 35300 | | 5.4 | | | | |
| hnRNP R | 2697103 | PF8 | 49100 | 70943 | 7.3 | 8.23 | 1 | - | - |
| Nucleolin | 4885511 | PF5 | 18100 | 76344 | 5.2 | 4.59 | 4 | + | + |
| p54nrb | 1895081 | NF3 | 55900 | 54231 | 8.5 | 9.01 | 2 | + | - |
| | | PF3 | 52300 | | 8.1 | | | | |
| Rho GDI 2 | 1707893 | NF2 | 23100 | 22988 | 5.1 | 5.10 | - | + | - |
| | | PF2 | 22100 | | 6.2 | | | | |
| Splicing factor ASF-2 | 105294 | NF5 | 31400 | 31999 | 5.2 | 5.61 | 1 | + | - |
| Splicing factor SRp30c | 4506903 | PF7 | 27300 | 25542 | 8.6 | 8.70 | 1 | + | - |
| Transcription factor BTF3 | 29507 | NF6 | 19000 | 17699 | 7.7 | 6.85 | - | + | + |

| | | | | | | | | | |
|---|---|---|---|---|---|---|---|---|---|
| Known interactions with protein kinase CK2 are displayed with an asterisk *. The sign ^{#} indicates that hnRNP C1/C2 was identified in seven spots, three times in negative Fas and four times in positive Fas Jurkat T- cells. | | | | | | | | | |

**Table 1b**

| Summary of factors modified by apoptosis. Detailed characterization is given in Tables 2-6. | | | | |
|---|---|---|---|---|
| Group | Proteins | Localization | Modification during apoptosis | known function |
| hnRNPs | A/B | NC | | RNP motif |
| | A1 | TMNC | | known substrate, RNP motif |
| | A2/B1 | TMNC | | RNP motif |
| | A3 | N | | RNP motif |
| | C1/C2 | TMN | | known substrate, RNP motif |
| | D | NC | | RNP motif |
| | F | M | | RNP motif |
| | H | NC | | RNP motif |
| | I | N | | RNP motif |
| | K | NC | | RNP motif |
| | L | N | | RNP motif |
| | R | TN | | RNP motif |
| | JKTBP1 | N | | RNP motif |
| Splicing factors | SRp30c | TN | only in apoptotic cells; also in the nucleus | splicing factor, RNP motif |
| | P54nrb | TN | modified; processed | splicing factor or nuclear matrix protein, respectively, RNP motif |
| | SF2p33 (ASF-2) | TN | missing in the nucleus of apoptotic cells | splicing factor; for alternative splicing variant function in the splicing of the Caspase-2 was described, RNP motif |
| | SF SC35 | N | missing in the nucleus of apoptotic cells; extreme IP shift | splicing factor; for alternative splicing variant function in the splicing of the Caspase-2 was described, RNP motif |
| | NMP200 (rel. to SF PRP19) | N | missing in the nucleus of apoptotic cells | protein of the nuclear matrix; unknown function |
| | PTB-associated SF | C | Cytosol of apoptotic cells | splicing factor, RNP motif |
| Translation | 60S acidic ribosomal protein | MN | ribosomal protein; normal cell in the nucleus/ER; apoptotic cells in mitochondria; altered IP; probably by phosphorylation | known substrate |
| | EF-Tu | N | mitochondrial protein; found in the nucleus of apoptotic cells | translation |
| | EF-1 beta | N | missing in the nucleus of apoptotic cells | no function known for apoptosis |
| | EIF-5A | C | missing in the cytosol of apoptotic cells | cellular target of HIV type 1 Rev binding factor |
| Structural | Lamin B1 | NC | nucleus of apoptotic cells and as fragment in the cytosol of apoptotic cells | known substrate |
| | Lamin B2 | NC | nucleus of apoptotic cells and cytosol of apoptotic cells | known substrate |
| | Vimentin | N | nucleus of apoptotic cells | known substrate |
| | Beta-Tubulin | C | cytosol of apoptotic cells | known substrate |
| Signal transduction proteins | GAP SH3 binding protein | T | Lysate of apoptotic cells, presumably processed | Ras-Oncogene signal protein, RNP motif |
| | Rho GDI2 | TMNC | | known substrate, published |
| | cGMP-dependent protein kinase type I alpha | M | mitochondria of apoptotic cells; presumably processed | Ser/Thr kinase, unknown function |
| Chromatin | Nucleolin | TC | in the lysate of apoptotic cells, presumably processed | known substrate; multifunctional protein, chromatin structure, RNP motif |
| | Baf-57 | N | missing in the nucleus of apoptotic cells | regulator of the chromatin structure |
| | CAF-1 (RB b.p.) (WD-repeats) | N | missing in the nucleus of apoptotic cells | binds to retinoblastoma, patent on WD repeat applications |
| Transcription factor | BTF3 | TC | missing in the lysate of apoptotic cells | already published |
| | CBF-beta | N | in the nucleus of apoptotic cells | binds to enhancers of murine leukaemia virus, Polioma virus, TCR etc., known alteration in the case of acute myeloid leukaemia |
| Proteasome | 26S protease subunit 12 | N | in the nucleus of apoptotic cells | regulatory subunit of the proteasome |
| | proteasome subunit C8 | C | in the cytosol of apoptotic cells | regulatory subunit of the proteasome |
| | Tat binding protein-1 | C | missing in the cytosol of apoptotic cells | regulatory subunit of the proteasome; HIV type 1 Tat binding protein |
| Mitochondrial | Isocitrate dehydrogenase | N | in the nucleus of apoptotic cells; maybe shortened | citrate cycle |
| | AOP-1 | N | N-terminal shortened, in the nucleus of apoptotic cells | anti-oxidant |
| Miscellaneous | Nucleophosmin | N | missing in apoptotic cells | already published |
| | SYT interacting protein SIP | N | missing in apoptotic cells | unknown, altered in synovial sarcoma cells |
| | PA1-G | N | in the nucleus of apoptotic cells | acetylhydrolase |
| | CRHSP-24 | N | in the nucleus of apoptotic cells | substrate of calcineurin |
| | HCD2 | NC | in the nucleus of apoptotic cells, missing in the cytosol of apoptotic cells, maybe phosphorylated | interaction with amyloid β-peptide; Alzheimer's disease |
| | *GMP synthase* | *C* | *in the cytosol of* apoptotic cells | *synthesis of guanin* nucleotides, particularly GTP |
| | FUSE binding protein 1 | C | in the cytosol of apoptotic cells | activation of the far-upstream element of c-myc |
| | HDGF | C | missing in the cytosol of apoptotic cells | hepatoma-derived growth factor, mitogenic activity for fibroblasts |
| T = Total lysate | | | | |
| M = Mitochondria | | | | |
| N = Nucleus | | | | |
| C = Cytosol | | | | |

**Table 2**

| Prediction of cleavage sites for apoptosis-modified proteins found in the total cell lysate | | | | | | | | | |
|---|---|---|---|---|---|---|---|---|---|
| Protein | No. AA | Sequence coverage | Cleavage site | Start-end AA | Mass (kDa) | Mass found | pl | pl found | Putative cleaved sequence |
| hnRNP A1 | 371 | 15-178 | CT | 1-288 | 30.5 | 32.1 | 8.4 | 8.5 | GSYD |
| | | | | 1-314 | 32.9 | | 8.4 | | SYND |
| hnRNP | 351 | 102-350 | NT* | 49-353 | 31.6 | 29.9 | 8.9 | 9.3 | KLTD |
| A2/B1 | | | | 56-353 | 30.82 | | 9.2 | | VMRD |
| | | | | | 8.6 | | | | |
| | | | | 76-353 | | | 8.8 | | AEVD |
| HnRNP | 303 | 18-151 | - | 1-295 | 32.5 | 35.3 | 5.2 | 5.4 | EGED |
| C1/C2 | | | | 10-303 | 32.3 | | 5.0 | | NKTD |
| hnRNP R | 624 | 134-441 | CT | 1-463 | 52.1 | 49.1 | 5.9 | 7.3 | YPPD |
| | | | | 20-463 | 49.9 | | 6.5 | | EPMD+YPPD |
| Nucleolin | 706 | 458-624 | NT+CT | 454-628 | 19.4 | 18.1 | 5.0 | 5.2 | TEID+AMED |
| | | | | 454-632 | 19.8 | | 4.9 | | TEID+GEID |
| p54nrb | 471 | 76-336 | (CT*) | 1-422 | 49.2 | 52.3 | 8.4 | 8.1 | MMPD |
| Rho GDI 2 | 201 | 22-196 | NT | 22-196 | 20.9 | 22.1 | 6.2 | 6.2 | DELD |

| | | | | | | | | | |
|---|---|---|---|---|---|---|---|---|---|
| The asterisk * displays that the comparison of the PMF of negative- and positive Fas showed an additional intense peak of the negative Fas spot outside the covered sequence and confirms the cleavage site (Figure 3). In parenthesis means that the cleavage site could not clearly identified only by sequence coverage of the PMF of the positive Fas spot. | | | | | | | | | |

**Table 3**

| **Proteins of the total cell lysate** | | | | | | |
|---|---|---|---|---|---|---|
| **Spot** | **Protein** | **NCBI** | **Mr** | **Mr** | **pI** | **pI** |
| | | | **theor.** | **found** | **theor.** | **found** |
| **PF1** | **hnRNP C1/C2** | **4758544** | **31966** | **35300** | **5.10** | **5.3** |
| **PF2** | **RhoGDI 2** | **1707893** | **22988** | **22400** | **5.10** | **6.2** |
| **PF3** | **P54nrb** | **1895081** | **54231** | **52300** | **9.01** | **8.1** |
| **PF4** | **hnRNP A2/B1** | **4504447/ 133257** | **36006/** | **36300** | **8.67/8.97** | **9.6** |
| | | | **37429** | | | |
| **PF5** | **Nucleolin** | **4885511** | **76344** | **18100** | **4.59** | **5.2** |
| **PF6** | **hnRNP A1** | **133254** | **38846** | **35200** | **9.26** | **9.6** |
| **PF7** | **Splicing factor SRp30c** | **4506903** | **25542** | **27300** | **8.70** | **8.6** |
| **PF8** | **hnRNP R** | **2697103** | **70943** | **49100** | **8.23** | **7.3** |
| **PF9** | **Unknown**^{**1**} | - | - | **24900** | - | **5.3** |
| **NF1** | **hnRNP C1/C2** | **4758544** | **31966** | **36300** | **5.10** | **5.3** |
| **NF2** | **RhoGDI 2** | **1707893** | **22988** | **22400** | **5.10** | **6.4** |
| **NF3** | **P54nrb** | **1895081** | **54231** | **55900** | **9.01** | **8.5** |
| **NF4** | **hnRNP A2/B1** | **4504447/ 133257** | **36006/** | **35700** | **8.67/8.97** | **8.7** |
| | | | **37429** | | | |
| **NF5** | **Splicing factor 2p33 (ASF-2)** | **105294** | **31999** | **31400** | **5.61** | **5.2** |
| **NF6** | **Transcription factor BTF3** | **29507** | **17699** | **19000** | **6.85** | **7.7** |

| | | | | | | |
|---|---|---|---|---|---|---|
| ^{**1**} **: Peptide mass fingerprint of the tryptic digestion (internal name: PFC6D)** | | | | | | |

**Table 4**

| **Proteins of the cytosol** | | | | | | |
|---|---|---|---|---|---|---|
| **Spot** | **Protein** | **NCBI** | **Mr** | **Mr** | **pI** | **pI** |
| | | | **theor.** | **found** | **theor.** | **found** |
| Cpf1 | Beta-Tubulin | 135448 | 49759 | 49800 | 4.75 | 5.2 |
| Cpf2 | PTB-associated splicing factor | 4826998 | 76149 | 89000 | 9.45 | 8.7 |
| Cpf3 | PTB-associated splicing factor | **4826998** | **76149** | **76000** | **9.45** | **8.3** |
| **Cpf4** | **GMP synthase** | **4504035** | **76715** | **62100** | **6.42** | **6.9** |
| **Cpf5** | **FUSE binding protein 1** | **4503801** | **67534** | **65200** | **7.21** | **7.7** |
| **Cpf6** | **FUSE binding protein 1** | **4503801** | **67534** | **65400** | **7.21** | **7.8** |
| **Cpf7** | **hnRNP D** | **870749** | **38434** | **43100** | **7.61** | **7.4** |
| **Cpf8** | **hnRNP A/B** | **4758542** | **31233** | **39100** | **9.35** | **6.6** |
| **Cpf9** | **hnRNP A/B** | **4758542** | **31233** | **36200** | **9.35** | **7.6** |
| **Cpf10** | **hnRNP A2/B1** | **4504447** | **36006/** | **39000** | **8.67/8.97** | **9.6** |
| | | **/133257** | **37429** | | | |
| **Cpf11** | **hnRNP A2/B1** | **4504447 /133257** | **36006/ 37429** | **35700** | **8.67/8.97** | **8.4** |
| **Cpf12** | **hnRNP A1** | **133254** | **38846** | **35200** | **9.26** | **9.5** |
| **Cpf13** | **hnRNP A1** | **133254** | **38846** | **35200** | **9.26** | **9.6** |
| **Cpf14** | **Proteasome subunit C8** | **130859** | **28433** | **26600** | **5.19** | **5.2** |
| **Cpf15** | **Lamin B2** | **547822** | **59001** | **22400** | **5.87** | **5.8** |
| **Cpf16** | **Nucleolin** | **4885511** | **76344** | **19000** | **4.59** | **5.2** |
| **Cpf17** | **Lamin B1** | **5031877** | **66408** | **22400** | **5.11** | **5.0** |
| **Cpf18** | **RhoGDI 2** | **1707893** | **22988** | **22400** | **5.10** | **6.2** |
| **Cpf19** | **RhoGDI 2** | **1707893** | **22988** | **22400** | **5.10** | **6.4** |
| **Cpf20** | **hnRNP A1** | **133254** | **38846** | **32100** | **9.26** | **8.1** |
| **Cpf21** | **hnRNP A1** | **133254** | **38846** | **35200** | **9.26** | **9.6** |
| **Cnf1** | **hnRNP K** | **631471** | **51072** | **65500** | **5.14** | **5.2** |
| **Cnf2** | **hnRNP H** | **1710632** | **49229** | **54100** | **5.89** | **6.1** |
| **Cnf3** | **HDGF** | **4758516** | **26788** | **36100** | **4.70** | **4.7** |
| **Cnf4** | **Tat binding protein-1** | **4506211** | **45165** | **45100** | **5.31** | **5.0** |
| **Cnf5** | **RhoGDI 2** | **1707893** | **22988** | **23100** | **5.10** | **5.1** |
| **Cnf6** | **EIF-5A** | **4503545** | **16701** | **17000** | **5.08** | **5.3** |
| **Cnf7** | **Transcription factor BTF3** | **29507** | **17699** | **19000** | **6.85** | **7.7** |
| **Cnf8** | **HCD2** | **4758504** | **26923** | **24800** | **7.65** | **6.2** |

**Table 5**

| **Proteins of the nucleus** | | | | | | |
|---|---|---|---|---|---|---|
| **Spot** | **Protein** | **NCBI** | **Mr** | **Mr** | **pI** | **pI** |
| | | | **theor.** | **found** | **theor.** | **found** |
| Kpf1 | hnRNP R | 2697103 | 70943 | 49100 | 8.23 | 7.2 |
| Kpf2 | Isocitrate dehydrogenase | 4504575 | 46644 | 43100 | 8.32 | 8.2 |
| **Kpf3** | **Elongation factor Tu** | **4507733** | **49540** | **41400** | **7.26** | **7.0** |
| **Kpf4** | **26S proteasome regulatory chain 12** | **4506231** | **37060** | **38800** | **6.11** | **7.2** |
| **Kpf5** | **hnRNP C1/C2** | **4758544** | **31966** | **35300** | **5.10** | **5.3** |
| **Kpf6** | **hnRNP A2/B1** | **4504447/** | **36006/** | **30000** | **8.67/** | **9.0** |
| | | **133257** | **37429** | | **8.97** | |
| **Kpf7** | **Splicing factor SRp30c** | **4506903** | **25542** | **28300** | **8.74** | **8.6** |
| **Kpf8** | **PA1-G** | **4505587** | **25734** | **28500** | **6.33** | **6.3** |
| **Kpf9** | **HCD2** | **4758504** | **26923** | **24800** | **7.65** | **6.2** |
| **Kpf10** | **AOP-1** | **5802974** | **27692** | **22500** | **7.67** | **6.1** |
| **Kpf11** | **Rho GDI 2** | **1707893** | **22988** | **22400** | **5.10** | **6.2** |
| **Kpf12** | **Rho GDI 2** | **1707893** | **22988** | **22400** | **5.10** | **6.4** |
| **Kpf13** | **Rho GDI 2** | **1707893** | **22988** | **22100** | **5.10** | **6.4** |
| | | **2498753** | **21508** | | | |
| | **CBF-beta** | | | | **6.23** | |
| **Kpf14** | **CRHSP-24** | **4583307** | **15934** | **21300** | **8.41** | **8.8** |
| **Kpf15** | **Unknown**^{**2**} | **-** | **-** | **59100** | **-** | **8.3** |
| **Knf1** | **hnRNP K** | **631471** | **51072** | **65600** | **5.14** | **5.2** |
| **Knf2** | **Lamin B1** | **125953** | **66408** | **65600** | **5.11** | **5.2** |
| **Knf3** | **hnRNP K** | **631471** | **51072** | **65100** | **5.14** | **5.4** |
| **Knf4** | **Lamin B2** | **547822** | **59001** | **62800** | **5.87** | **5.4** |
| **Knf5** | **hnRNP K** | **631471** | **51072** | **59700** | **5.14** | **6.0** |
| **Knf6** | **NMP200 (related to splicing factor** | **5689738** | **55181** | **55500** | **6.14** | **6.4** |
| | **PRP19)** | | | | | |
| **Knf7** | **BAF57** | **4507089** | **46649** | **54700** | **4.85** | **4.9** |
| **Knf8** | **Vimentin** | **2119204** | **53651** | **56200** | **5.06** | **5.1** |
| **Knf9** | **CAF-1** | **422892** | **46158** | **53000** | **4.90** | **4.7** |
| **Knf10** | **hnRNP H** | **1710632** | **49229** | **50600** | **5.89** | **6.1** |
| **Knf11** | **Splicing factor 2p33 (ASF-2)** | **105294** | **31999** | **31400** | **5.61** | **5.2** |
| **Knf12** | **hnRNP H** | **1710632** | **49229** | **42100** | **5.89** | **6.6** |
| **Knf13** | **Splicing factor 2p33 (ASF-2)** | **105294** | **31999** | **31400** | **5.61** | **5.1** |
| **Knf14** | **hnRNP A/B** | **4758542** | **31233** | **39000** | **9.35** | **6.4** |
| **Knf15** | **hnRNP C1/C2** | **4758544** | **31966** | **36300** | **5.10** | **5.0** |
| **Knf16** | **Nucleophosmin** | **114762** | **32575** | **35300** | **4.64** | **4.8** |
| **Knf17** | **60S acidic ribosomal protein** | **4506667** | **34273** | **33500** | **5.72** | **5.8** |
| **Knf18** | **JKTBP1** | **2780748** | **33589** | **36100** | **6.85** | **6.3** |
| **Knf19** | **JKTBP1** | **2780748** | **33589** | **36100** | **6.85** | **6.6** |
| **Knf20** | **SYT interacting protein SIP** | **5454064** | **69492** | **73000** | **9.68** | **9.0** |
| **Knf21** | **hnRNP L** | **4557645** | **60187** | **67400** | **6.65** | **7.4** |
| **Knf22** | **hnRNP I** | **131528** | **57221** | **53700** | **9.22** | **8.5** |
| **Knf23** | **hnRNP I** | **131528** | **57221** | **53900** | **9.22** | **8.6** |
| **Knf24** | **P54nrb** | **1895081** | **54231** | **54000** | **9.01** | **9.2** |
| **Knf25** | **hnRNP D** | **870749** | **38434** | **44100** | **7.61** | **6.9** |
| **Knf26** | **hnRNP A1** | **133254** | **38846** | **35200** | **9.26** | **9.6** |
| **Knf27** | **hnRNP A2/B1** | **4504447/** | **36006/** | **35700** | **8.67/** | **8.4** |
| | | **133257** | **37429** | | **8.97** | |
| **Knf28** | **hnRNP A3** | **1710627** | **39686** | **39000** | **8.74** | **9.6** |
| **Knf29** | **hnRNP A2/B1** | **4504447/** | **36006/** | **36400** | **8.67/** | **9.7** |
| | | **133257** | **37429** | | **8.97** | |
| **Knf30** | **hnRNP A3** | **1710627** | **39686** | **36400** | **8.74** | **8.3** |
| **Knf31** | **hnRNP A2/B1** | **4504447/** | **36006/** | **36200** | **8.67/** | **8.9** |
| | | **133257** | **37429** | | **8.97** | |
| **Knf32** | **hnRNP A2/B1** | **4504447/** | **36006/** | **35700** | **8.67/** | **8.2** |
| | | **133257** | **37429** | | **8.97** | |
| **Knf33** | **Splicing factor SC35** | **539663** | **25476** | **28100** | **11.86** | **5.1** |
| **Knf34** | **RhoGDI 2** | **1707893** | **22988** | **23100** | **5.10** | **5.1** |
| **Knf35** | **RhoGDI 2** | **1707893** | **22988** | **21300** | **5.10** | **4.8** |
| **Knf36** | **Elongation factor 1-beta** | **4503477** | **24763** | **24800** | **4.50** | **4.5** |
| **Knf37** | **Unknown**^{**1**} | **-** | **-** | **61300** | **-** | **6.6** |

| | | | | | | |
|---|---|---|---|---|---|---|
| ^{**1**} **: Peptide mass fingerprint of the tryptic digestion and MS/MS-spectrum of mass 1649,76 dalton with the sequence TPGT(F/Mox)E (internal name: KNFE3)** | | | | | | |
| ^{**2**} **: Peptide mass fingerprint of the tryptic digestion (internal name: KPF1)** | | | | | | |

**Table 6**

| **Proteins of the mitochondria** | | | | | | |
|---|---|---|---|---|---|---|
| **Spot** | **Protein** | **NCBI** | **Mr** | **Mr** | **pI** | **pI** |
| | | | **theor.** | **found** | **theor.** | **found** |
| Mpf1 | hnRNP F | 4836760 | **45672** | 47600 | **5.38** | **5.2** |
| **Mpf2** | **hnRNP C1/C2** | **4758544** | **31966** | **35300** | **5.10** | **5.3** |
| **Mpf3** | **CGMP-dependent protein kinase type I** | **6225588** | **76364** | **45300** | **5.74** | **6.2** |
| | **alpha** | | | | | |
| | | **4504453** | **51072** | | **5.14** | |
| | **hnRNP K** | | | | | |
| **Mpf4** | **60S acidic ribosomal protein** | **4506667** | **34273** | **33300** | **5.72** | **6.1** |
| **Mpf5** | **hnRNP A2/B1** | **4504447/ 133257** | **36006/** | **36300** | **8.67/8.97** | **9.6** |
| | | | **37429** | | | |
| **Mpf6** | **hnRNP A2/B1** | **4504447/ 133257** | **36006/** | **35700** | **8.67/8.97** | **8.7** |
| | | | **37429** | | | |
| **Mpf7** | **hnRNP A1** | **133254** | **38846** | **35200** | **9.26** | **9.6** |
| **Mpf8** | **hnRNP A1** | **133254** | **38846** | **35200** | **9.26** | **9.7** |
| **Mpf9** | **RhoGDI 2** | **1707893** | **22988** | **22100** | **5.10** | **6.2** |
| **Mpf10** | **RhoGDI 2** | **1707893** | **22988** | **22100** | **5.10** | **6.4** |

## Claims

1. A method for characterizing and/or identifying apoptosis-modified proteins comprising the steps:
(a) providing a first extract and a second extract comprising soluble proteins, wherein said first extract is from a cell without apoptosis induction and said second extract is from a cell after apoptosis induction,
(b) separating said first and second extracts by two-dimensional gel electrophoresis, wherein first and second proteome patterns each comprising a plurality of protein species are obtained,
(c) comparing said first and second proteome patterns and
(d) characterizing and/or identifying apoptosis-modified protein species.

2. The method of claim 1, wherein after apoptosis induction substantially no synthesis of new proteins has been allowed.

3. The method of claim 2, wherein the protein biosynthesis has been substantially blocked by an inhibitor.

4. The method of claim 2 or 3, wherein apoptosis induction has been carried out for a period of time which is too short to allow a substantial synthesis of new proteins.

5. The method of any one of claims 1-4, wherein said two-dimensional gel electrophoresis comprises (i) separation in a first dimension according to the isoelectric point and (ii) separation in a second dimension according to size.

6. The method of any one of claims 1-5, wherein the apoptosis-modified protein species are selected from protein species which (i) are located at different positions on the two-dimensional gels from the first and second extracts and/or (ii) have a different intensity on the two-dimensional gels from the first and second extracts.

7. The method of any one of claims 1-6, wherein the protein species are **characterized by** peptide fingerprinting.

8. The method of claim 7, wherein the peptides are **characterized by** mass spectrometry and/or at least partial sequencing.

9. The method of any one of claims 1-8, wherein said cell is a mammalian cell.

10. The method of claim 9, wherein said cell is a human cell.

11. The method of claim 9 or 10, wherein said cell is a T-cell.

12. The method of claim 11, wherein said T-cell is the T-cell line Jurkat E6 (ATCC TIB 152).

13. The method of any one of claims 1-12, wherein the apoptosis is induced by an anti-Fas antibody.

14. The method of any one of claims 1-13, wherein the apoptosis-modified protein species are selected from heterogeneous nuclear ribonucleoproteins, splicing factors, translation factors, structural proteins, signal transduction proteins, chromatin associated proteins, transcription factors, proteasome subunits, mitochondrial proteins, nucleophosmin, SYT interacting protein SIP, PA1-G, CRHSP-24, HCD2, GMP synthase, FUSE binding protein 1, HDGF, PFC6D1, KPF1 and KNFE3 having the partial sequence TPGT (F/Mox)E or a partial sequence derived therefrom by substitution of one or more amino acids.

15. The method of any one of claims 1-14 further comprising
(e) determining if the apoptosis-modified proteins are present in subjects suffering from apoptosis-associated diseases.

16. Proteome from an apoptotic T-cell or a compartment thereof consisting of a pattern of individual proteins obtainable by the method of any one of claims 1-15.

17. The proteome of claim 16 containing the proteins as shown in Table 1 or at least a part thereof.

18. Apoptosis-modified protein selected from heterogeneous nuclear ribonucleoproteins, splicing factors, translation factors, structural proteins, signal transduction proteins, chromatin associated proteins, transcription factors, proteasome subunits, mitochondrial proteins, nucleophosmin, SYT interacting protein SIP, PA1-G, CRHSP-24, HCD2, GMP synthase, FUSE binding protein 1, HDGF, PFC6D1, KPF1 and KNFE3 having the partial sequence TPGT (F/Mox)E or a partial sequence derived therefrom by substitution of one or more amino acids.

19. Apoptosis-modified protein selected from the proteins as shown in Table 1.

20. Use of a proteome of claim 16 or 17 or a protein of claims 18 or 19 as target for the diagnosis, prevention or treatment of apoptosis-associated diseases or in a method for identifying apoptosis modulators.
